(19) Europäisches Patentamt / European Patent Office / Office européen des brevets

(11) **EP 2 545 963 A1**

(12) **EUROPEAN PATENT APPLICATION**

(43) Date of publication:
**16.01.2013 Bulletin 2013/03**

(51) Int Cl.:
***A61N 7/02*** *(2006.01)*

(21) Application number: **12174298.5**

(22) Date of filing: **29.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.07.2011 KR 20110070029**

(71) Applicant: **Samsung Electronics Co., Ltd.**
**Suwon-si, Gyeonggi-do, 443-742 (KR)**

(72) Inventors:
- **Park, Jun-ho**
  **Yongin-si**
  **Gyeonggi-do (KR)**
- **Lee, Hyoung-ki**
  **Yongin-si**
  **Gyeonggi-do (KR)**
- **Lee, Ho-taik**
  **Yongin-si**
  **Gyeonggi-do (KR)**
- **Ahn, Min-su**
  **Yongin-si**
  **Gyeonggi-do (KR)**
- **Park, Ji-young**
  **Yongin-si**
  **Gyeonggi-do (KR)**

(74) Representative: **Land, Addick Adrianus Gosling et al**
**Arnold & Siedsma**
**Sweelinckplein 1**
**2517 GK Den Haag (NL)**

Remarks:
The reference to the drawing no. 11 is deemed to be deleted (Rule 56(4) EPC).

(54) **Focused ultrasound therapy apparatus controlling a focal point**

(57) A method of controlling a focus in a focused ultrasound therapy apparatus, the method including receiving an target area to which ultrasound is radiated to remove a lesion; determining a path through which the focus moves in the target area, depending on a form of the target area; and forming the focus on the determined path and then radiating ultrasound to the target area.

FIG. 1

# EP 2 545 963 A1

**Description**

Embodiments relate to a focused ultrasound therapy apparatus used in noninvasive surgery for the local treatment of a tumor.

Along with the progress of medical science, recently, noninvasive surgery as well as minimum invasive surgery has been used for the local treatment of a tumor. High intensity focused ultrasound (HIFU) from among the noninvasive surgery methods has been widely used since ultrasound is harmless to the human body. HIFU is a treatment method of necrotizing a lesion by focusing and radiating high intensity ultrasound to the lesion in the human body. Ultrasound focused and radiated to the lesion is converted into thermal energy that causes coagulating necrosis of the lesion and blood vessels due to a temperature increase of a portion to which ultrasound is radiated. Since the temperature is raised instantly, it is possible to effectively remove only the radiated portion while preventing heat from diffusing to surrounding areas of the radiated portion.

According to an aspect of one or more embodiments, there are provided focused ultrasound therapy apparatuses for reducing a treatment time when removing lesions of various forms in the human body.

According to an aspect of one or more embodiments, there are provided methods of controlling a focal point in the focused ultrasound therapy apparatuses.

According to an aspect of one or more embodiments, there is provided a method of controlling a focus in a focused ultrasound therapy apparatus for radiating ultrasound to remove a lesion, the method includes: receiving s target area to which ultrasound is radiated to remove the lesion; determining a path along which the focus moves in the target area, depending on a form of the target area; and forming the focus on the determined path and then radiating ultrasound to the target area.

The method may further include determining a size of the focus to be used for ultrasound radiation, wherein the determining of the path includes determining the path along which the focus moves in the target area, depending on the determined size of the focus.

The determining of the path may include setting a path having a form which is the same as a boundary line of the target area.

The determining of the path may include setting more than two paths, and the radiating of ultrasound may include forming focuses having sizes different from each other on paths different from each other and then radiating ultrasound.

According to an aspect of one or more embodiments, there is provided a method of controlling a focus in a focused ultrasound therapy apparatus for radiating ultrasound in a target area to remove a lesion, the method includes: determining more than one path along which the focus moves in the target area, depending on a form of the target area; forming the focus along each determined path, and then radiating the ultrasound to the target area, wherein a size of the focus on at least one path is different from a size of the focus on at least one other path.

The radiating of ultrasound may include forming a multi-focus and then radiating ultrasound.

The radiating of ultrasound may include forming a smaller focus in a portion closer to an edge of the target area and then radiating ultrasound. The focus in the portion close to the edge of the target area may be smaller than the focus in another portion further removed from the edge of the target area.

The radiating of ultrasound may include: calculating a radiation time of ultrasound; and radiating ultrasound to the target area for the calculated radiation time.

The calculating of the radiation time may include estimating a temperature change of the target area depending on time, in a case of radiating ultrasound to the target area, by using a distance from a point where ultrasound is generated from the target area, a mass composition of an organ, and a blood diffusion degree in the body, calculating a time during which an energy transmitted to the target area reaches a value higher than a critical value by using the estimated temperature change, and then determining the calculated time as the radiation time.

The forming the focus on the determined path and then radiating of ultrasound may include: determining positions where the focus is formed on the determined path; and determining an order of the determined positions, forming the focus depending on the determined order, and then radiating ultrasound.

The receiving of the target area may include, after determining whether it is possible to control the focus in the target area, receiving another target area if it is determined that the control of the focus is impossible.

According to an aspect of one or more embodiments, there is provided a focused ultrasound therapy apparatus for radiating ultrasound to remove a lesion, the apparatus includes: a target area input unit to receive a target area to which ultrasound is radiated to remove the lesion; a path determination unit to determine a path along which a focus moves in the target area, depending on a form of the target area; an ultrasound converter to convert an electrical signal and then to generate ultrasound; and a focus control unit (focus controller) to control the ultrasound converter so as to form the focus on the determined path and then radiate ultrasound to the target area.

The path determination unit may determine the path along which the focus moves in the target area, depending on a size of the focus to be used for ultrasound radiation.

The path determination unit may set a path having a form which is the same as a boundary line of the target area.

The path determination unit may set more than two paths, and the focus control unit may control the ultrasound converter so as to form focuses having sizes different from each other on paths different from each other and then radiate ultrasound.

According to an aspect of one or more embodiments, there is provided an apparatus for radiating ultrasound in a target area to remove a lesion, the apparatus includes a path determination unit to set more than one path along which a focus moves in the target area, depending on a form of the target area; and a focus controller to control an ultrasound converter to form the focus along each predetermined path and to then radiate ultrasound to the target area, wherein a size of the focus on at least one path is different from a size of the focus on at least one other path.

The focus control unit may control the ultrasound converter so as to form a multi-focus and then radiate ultrasound.

The focus control unit may control the ultrasound converter so as to form a smaller focus in a portion closer to an edge of the target area than the focus in another portion farther removed from the edge of the target area and then radiate ultrasound.

The apparatus may further include a radiation time calculation unit (radiation time calculator) to calculate a radiation time of ultrasound, wherein the focus control unit controls the ultrasound converter so as to radiate ultrasound to the target area for the radiation time calculated in the radiation time calculation unit.

The radiation time calculation unit may estimate a temperature change of the target area depending on time, in the case of radiating the ultrasound to the target area, by using a distance from a point, in which the ultrasound is generated, from the target area, an organization composition of an organ, and a blood diffusion degree in the body, may calculate a time during which an energy transmitted to the target area reaches a value higher than a critical value by using the estimated temperature change, and then may determine the calculated time as the radiation time.

The focus control unit may control the ultrasound converter so as to determine positions where the focus is formed on the determined path, determine an order of the determined positions, form the focus depending on the determined order, and then radiate ultrasound.

The target area input unit may receive another target area if it is determined that a control of the focus is impossible, after determining whether it is possible to control the focus in the target area.

According to one or more embodiments of focused ultrasound therapy apparatus (es) and the method of controlling a focal point in the focused ultrasound therapy apparatus, it is possible to remove lesions of various forms by determining a path through which a focus will move in an target area, depending on a form of an input target area, and forming the focus on the determined path and then radiating ultrasound. In addition, it is possible to reduce a treatment time by previously determining a size of focus to be used, a radiation time of ultrasound, and a position and order in which the focus will be formed on the path, and then radiating ultrasound according to the determined elements.

According to another aspect of one or more embodiments, there is provided at least one non-transitory computer readable medium storing computer readable instructions to implement methods of one or more embodiments.

## BRIEF DESCRIPTION OF THE DRAWINGS

These and/or other aspects will become apparent and more readily appreciated from the following description of embodiments, taken in conjunction with the accompanying drawings in which:

FIG. 1 is a diagram illustrating an example in which a focused ultrasound therapy apparatus according to an embodiment;

FIG. 2 is a diagram illustrating an example of a target area input to the focused ultrasound therapy apparatus according to an embodiment;

FIG. 3 is a diagram illustrating a path, through which a focus will move, and a position and order, in which the focus will be formed, in an target area;

FIG. 4 is a diagram illustrating an example in which a multi-focus is formed in a target area;

FIG. 5 is a diagram illustrating a method of determining a path along which a focus will move in a target area;

FIG. 6 is a diagram illustrating a path along which a focus will move and a position and order in which the focus is formed, in a target area, according to an embodiment; and

FIGS. 7 through 11 are flowcharts for explaining a method of controlling a focus in a focused ultrasound therapy apparatus, according to an embodiment.

## DETAILED DESCRIPTION

Embodiments will now be described more fully with reference to the accompanying drawings. In the following description, well-known functions or constructions are not described in detail if it is determined that they would obscure the disclosure due to unnecessary detail.

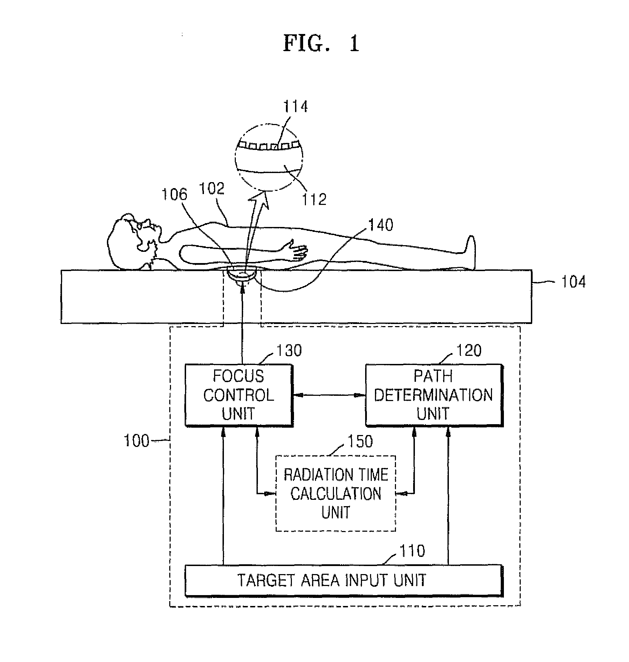

FIG. 1 is a diagram illustrating an example in which a focused ultrasound therapy apparatus 100 according to an embodiment is actually used. Referring to FIG. 1, the focused ultrasound therapy apparatus 100 may include a target area input unit 110, a path determination unit 120, a focus control unit (focus controller) 130, and an ultrasound converter 140.

As illustrated in FIG. 1, the ultrasound converter 140 may be installed inside a bed 104 on which a subject 102 is lying and may remove a lesion by radiating ultrasound to a specific portion inside the body of the subject 102. A gel pad 106 may be positioned between the subject 102 and the bed 104 to support transmission of ultrasound. In addition, the ultrasound converter 140 may radiate ultrasound while changing a position of a focus, namely a focal point in a predetermined target area, or may form a multi-focus, by controlling a phase of ultrasound generated by a plurality of elements 114 arranged in a round support plate 112 which has a concave shape. Since the ultrasound converter 140 is generally used in a focused ultrasound therapy apparatus of a phase-controlled type, a detailed description thereof is omitted. Below, an operation of the focused ultrasound therapy apparatus 100 is described.

An operation of a focused ultrasound therapy apparatus starts when a target area input unit receives a target area to which ultrasound will be radiated. The target area is an area to which ultrasound may be radiated by moving a position of a focus through a phase control of the ultrasound converter 140. Thus, the size of the target area in which the focus may be moved through the phase control is limited, and recently, a target area with a size of about 10 through about 20 millimeter (mm) has been used. Thus, in order to remove a lesion which is larger than a realizable target area, the lesion is divided into a plurality of target areas, and then, ultrasound is radiated to the plurality of target areas. However, in general, since a shape (for example, a round shape) and size of the target area which may be set for each focused ultrasound therapy apparatus is predetermined, it is impossible to variously set the target area depending on a form of the lesion. That is, it is impossible to effectively treat lesions of various forms, and this problem seriously occurs at an edge portion of the lesion in particular, which causes an increase of the treatment time.

However, the focused ultrasound therapy apparatus 100 according to an embodiment may receive a target area of which a form is freely determined by a user operating the apparatus, and may determine a path, along which a focus will move, depending on a form of the target area and then may perform ultrasound radiation. Thus, it is possible to effectively treat lesions of various forms. In addition, the target area input unit 110 may determine whether the target area received from the user is an area where a focus may be controlled, and receive again a new target area in the case where the target area is an area where a focus may not be controlled. The target area input to the target area input unit 110 is transmitted to the path determination unit 120 and the focus control unit 130 and is used to determine a path along which a focus will move and a size of the focus to be used.

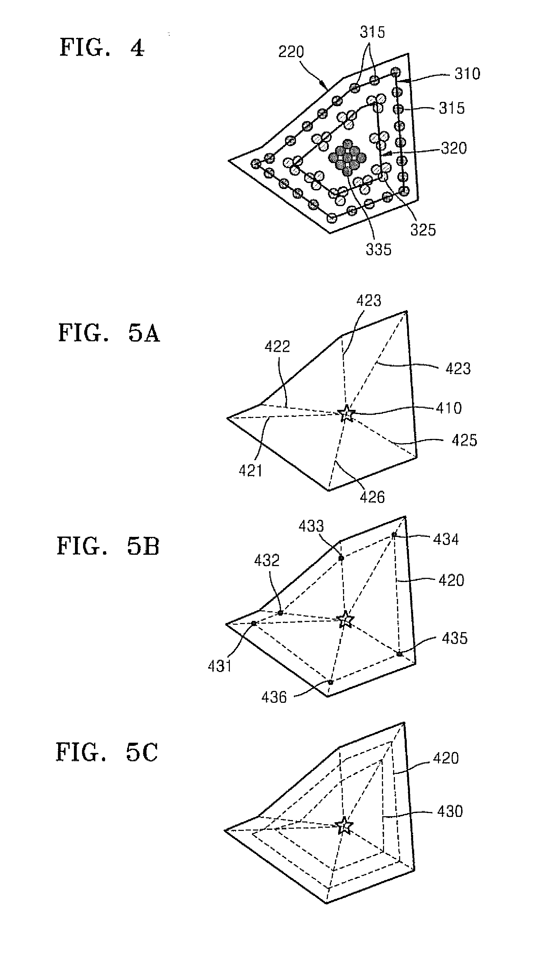

The path determination unit 120 determines the path along which the focus will move depending on a form of the received target area. In detail, the path determination unit 120 may determine the path so that a trajectory of the path is the same as or a similar to a boundary line of the target area. For example, if the target area has a lozenge form, the path determination unit 120 may determine the path in the lozenge form. It is possible to determine a plurality of paths which have a form similar to that of the target area and are reduced in different ratios from each other. It is also possible to determine a plurality of paths which have a form and a center of gravity, which are the same as those of the target area, and are reduced in different ratios from each other. A detailed explanation of the path determination is described with regard to FIG. 5 below. The path determination unit 120 may transmit the determined path to the focus control unit 130 to enable the focus control unit 130 to radiate ultrasound while moving a focus along the determined path.

The focus control unit 130 may control the ultrasound converter 140 to radiate the ultrasound by forming a focus on a target area according to the received target area and predetermined path. The focus control unit 130 may determine a size of the focus to be used depending on the determined path, and may determine different sizes of focuses to be used in the case of a plurality of paths different from each other. For example, it is possible to reduce the treatment time by using a large focus in a center of the target area, and it is possible to precisely radiate ultrasound by using a smaller focus in a portion closer to an edge of the target area. The focus control unit 130 may control the ultrasound converter 140 to allow it to form a multi-focus, and may also control a size of the multi-focus and a position where the multi-focus is formed by controlling a phase of ultrasound generated by each of the elements 114 of the ultrasound converter 140. In addition, the focus control unit 130 may determine a position where focuses are formed on the paths determined in the path determination unit 120, and a formation order of the focuses, and thus, may control the ultrasound converter 140 to allow it to perform ultrasound radiation. In addition, if the focus control unit 130 determines a size of the focus to be used and then transmits the size of the focus to the path determination unit 120, the path determination unit 120 may determine a path along which the focus will move based on the determined size of the focus and a form of the target area.

A focused ultrasound therapy apparatus 100 according to an embodiment may further include a radiation time calculation unit (radiation time calculator) 150. If the radiation time calculation unit 150 previously calculates the radiation time of ultrasound to be radiated to the target area, the focus control unit 130 may control the ultrasound converter 140 to allow it to radiate ultrasound for the calculated radiation time in order to reduce the treatment time. The radiation time calculation unit 150 calculates a minimum time necessary to necrotize a lesion in the target area by using the following method.

First, if a path, along which a focus will move, and a size of the focus to be used are determined, it is possible to obtain a temperature change depending on time in the target area in the case of radiating ultrasound to the target area by using the Pennes' bioheat transfer equation 1 below.

Equation 1

$$\rho C_t \frac{\partial T(\bar{r},t)}{\partial t} = k\nabla^2 T(\bar{r},t) - W_b C_b (T(\bar{r},t) - T_a) + \alpha f \frac{|\rho(\bar{r},t)|^2}{\rho c}$$

In equation 1, "$\rho$" is the density of the lesion in the target area, "$C_t$" is the specific heat of the lesion, "k" is the thermal conduction ratio, "$W_b$" is blood perfusion, "$C_b$" is the specific heat of blood, "$T_a$" is the temperature of blood, "$\alpha$" is a coefficient of reduction of lesion, "f" is the frequency of ultrasound, "p" is the pressure of ultrasound, and "c" is the speed of ultrasound. If a distance from the ultrasound converter 140 generating ultrasound to the target area is provided, it is possible to obtain a temperature change depending on the time to the target area by using equation 1.

If the temperature change depending on the time to the target area is obtained, it is possible to calculate a radiation time necessary for necrosis of a lesion mass by using the Sapareto and Dewey equation 2 below.

Equation 2

$$t_{43} = \sum_{t=0}^{t=t_f} R^{(43-\bar{T})} \Delta t$$

In equation 2, "$\bar{T}$" is an average temperature of the target area during a radiation time, "R" is value that changes according to "$\bar{T}$", and is equal to 0.5 if "$\bar{T}$" is more than 43 degrees Celsius, and is equal to 0.25 if "$\bar{T}$" is less than 43 degrees Celsius, and "$t_{43}$" is a thermal dose, and the unit of "$t_{43}$" is cumulative equivalent minutes (CEM). If "$t_{43}$" is 240 CEM, it may be determined that the lesion has necrotized. For example, it is determined that the lesion has necrotized if 240 minutes have passed at an average temperature of 43 degrees Celsius. Thus, if "$t_f$" is calculated so that the "$t_{43}$" becomes 240CEM, a value of the "$t_f$" is the radiation time necessary for necrosis of the lesion mass.

By the above method, it is possible to reduce the treatment time by calculating a necessary minimum radiation time via the radiation time calculation unit 150 and controlling the ultrasound converter 140 via the focus control unit 130 so as to radiate ultrasound only during the calculated time.

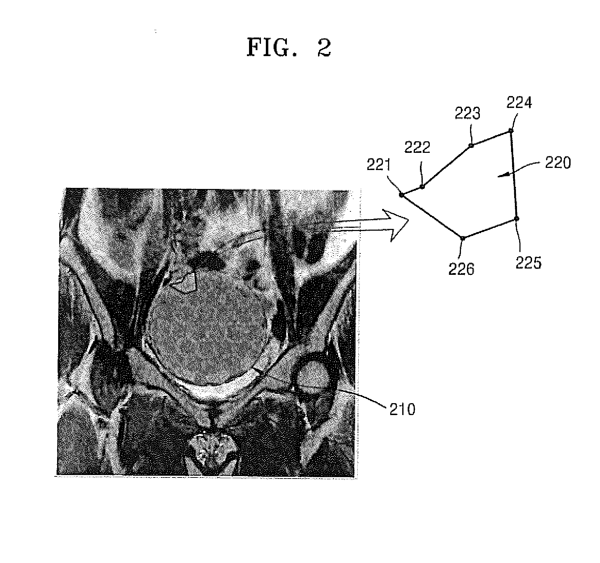

FIG. 2 is a diagram illustrating an example of a target area 220 input to the focused ultrasound therapy apparatus according to an embodiment. Referring to FIG. 2, the target area 220, to which ultrasound is radiated, is set on a lesion 210 to be removed. It is possible to effectively remove lesions of various forms by determining a form of the target area 220 depending on a form of the lesion 210. A user operating the focused ultrasound therapy apparatus may set the target area by using various methods. For example, if the user designates a plurality of points 221 through 226, it is possible to set the inner area of a polygon formed by connecting the designated points as the target area. Since the target area should be set in a form in which the focus control unit 130 may control a focus, the target area input unit 110 determines whether it is possible to control the focus in the input target area, and receives a new target area if this is not possible. The target area 220 illustrated in FIG. 2 is an example of the lesion 210, and a plurality of target areas may be formed on the lesion 210 by using a similar method.

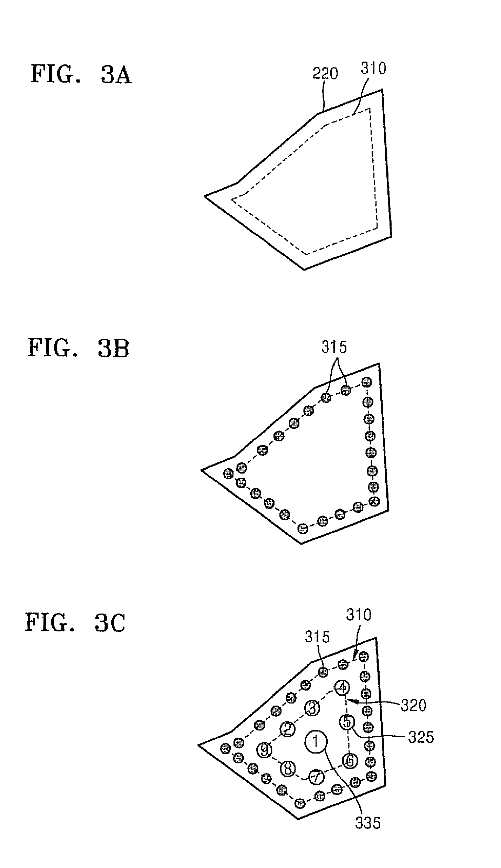

FIGS. 3 through 6 are diagrams for explaining a method of controlling a focus in the focused ultrasound therapy apparatus, according to an embodiment. A method of determining a path along which a focus will move, a size of the focus to be used, a position and order in which the focus will be formed, depending on input target area, is described below with reference to FIGS. 3 through 6.

FIG. 3 is a diagram illustrating the path, along which the focus will move, and the position and order in which the focus will be formed in the target area. In FIG. 3A, a focus movement path 310 having the same form as a target area 220 is set in the target area 220. In FIG. 3B, positions and forms of focuses 315 formed on the focus movement path 310 are illustrated. The focuses are formed at the positions indicated on the path 310, and thus, the focuses may be formed at these positions according to a predetermined order. The number of the focuses to be formed on the path 310 is determined according to the size of a focus to be used. If a larger focus is used, the treatment time is reduced since the number of focuses formed on the same path is decreased, but radiation accuracy of ultrasound is low. Thus, it is possible to reduce the treatment time by forming a larger focus in the center of the target area 220, where relatively smaller accuracy is required compared to an edge of the target area 220, and by forming a smaller focus in a portion closer to the edge of the target area 220. In FIG. 3C, two paths 310 and 320 are set in the target area 220, and focuses 315 and 325, which are respectively formed on the focus movement paths 310 and 320, and a focus 335, which is formed at the center of gravity of the target area 220, are illustrated. In addition, in FIG. 3C, the order in which the focuses are formed is indicated by using numbers in the focus 335 formed at the center of gravity and the focuses 325 formed on the inside path 320. In other words, since a number indicated in the focus 335 formed at the center of gravity of the target area 220 is "1", the focus 335 is formed first. In addition, focuses indicated by numbers 2, 3, 4, and the like are sequentially formed. The order in which the focuses are formed may be freely set for convenience of control, depending on the circumstances.

FIG. 4 is a diagram illustrating an example in which a multi-focus is formed in the target area 220. Referring to FIG. 4, a size of focus is adjusted through a method of forming the multi-focus. The size of the multi-focus 335 which is formed at the center of gravity of the target area 220 is the largest, and the sizes of the focuses 315 which are formed on the outside path 310 are smaller than those of the focuses 325 formed on the inside path 320. In this manner, it is possible to form a multi-focus of various forms through a phase control of each element of the ultrasound converter.

FIG. 5 is a diagram illustrating a method of determining a path along which a focus will move in the target area 220. First, the center point 410 of gravity of the target area 220 is obtained. In FIG. 5A, the center point 410 of gravity of the target area 220 and segments 421 through 426 which connect the center point 410 to vertexes 221 through 226 of the target area 220 are illustrated. Next, as shown in FIG. 5B, points 431 through 436, which are positioned on the segments 421 through 426 respectively and divide distances from the vertexes 221 through 226 of the target area 220 to the center point 410 by a constant ratio, are obtained. The ratio used for dividing the distances may be properly determined in consideration of the size along through which the focus will move is set by connecting the points 431 through 436 to each other. In FIG. 5C, the case where an additional path 430 besides the path 420 is further set by using the same method is illustrated.

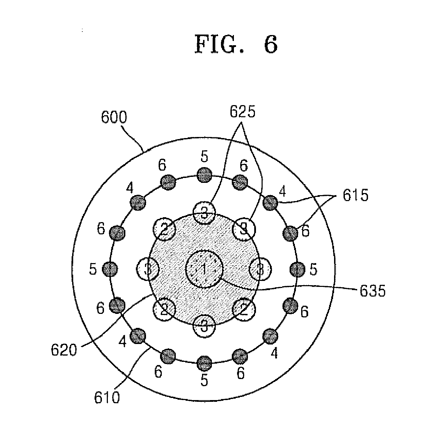

FIG. 6 is a diagram illustrating a path through which a focus will move and a position and order in which the focus is formed, in a target area, according to an embodiment. As illustrated in FIG. 6, the target area 600 may be set with a round form. In the target area 600 of the round form, a plurality of focus movement paths 610 and 620, which have center points of gravity which are the same as that of the target area and of which forms are round forms, may be set. With respect to sizes of focuses to be used, a size of a focus 635 which is formed at the center point of gravity of the target area 600 is the largest, and the sizes of the focuses 615 which are formed on the outside path 610 are the smallest. Focuses 625 are located on a focus path 620. A number indicated in each focus is an order in which each focus is formed. That is, the focus 635, which is formed at the center point of gravity in which "1" is indicated, is formed first and then focuses in which "2" is indicated, from among focuses which are formed on the inside path 620, are formed. A plurality of focuses in which the same number is indicated exist, which indicates that these focuses are formed simultaneously. This is because it is possible to control the ultrasound converter so as to simultaneously form the plurality of focuses in a symmetrical manner. Thus, it is possible to reduce the treatment time by controlling the ultrasound converter so as to simultaneously form the plurality of focuses.

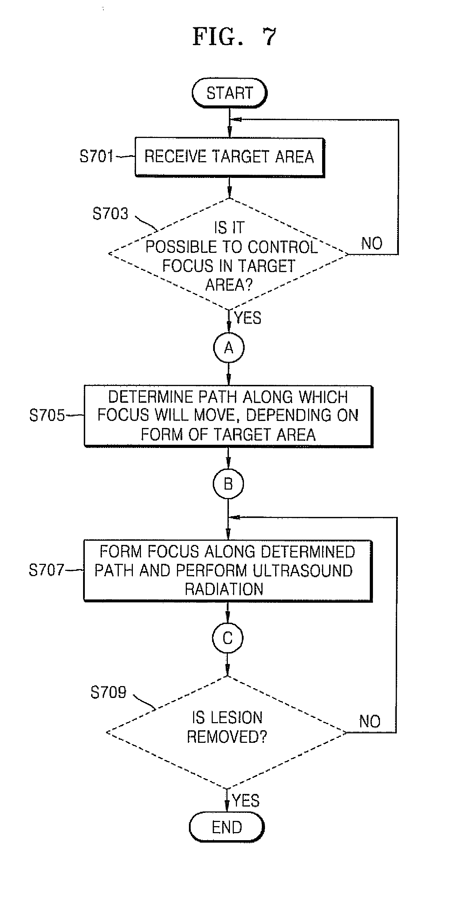

FIGS. 7 through 11 are flowcharts for explaining a method of controlling a focus in the focused ultrasound therapy apparatus according to an embodiment.

Referring to FIG. 7, first, a target area, to which ultrasound will be radiated, is received (operation S701). The target area is an area to which ultrasound may be radiated by moving a position of a focus through a phase control of the ultrasound converter 140. Thus, there is a limit regarding the size of the target area where a position of a focus may be moved through the phase control, and, recently, the target area has been embodied with a size of about 10 through about 20 millimeter (mm). Thus, in order to remove a lesion mass which is larger than a realizable target area, the lesion mass is divided into a plurality of target areas and then ultrasound is radiated to the plurality of target areas. However, in general, since a shape (for example, a round shape) and size of the target area which may be set for each focused ultrasound therapy apparatus is predetermined, it is impossible to variously set the target area depending on a form of the lesion. That is, it is impossible to effectively treat lesions of various forms, and this problem seriously occurs at an edge portion of the lesion in particular and thus cause an increase of the treatment time. However, in a method of controlling a focus in the focused ultrasound therapy apparatus according to an embodiment, a target area, a form of which is freely determined by a user operating the apparatus, may be received, and it is possible to along through which a focus will move, depending on a form of the target area and then performing ultrasound radiation.

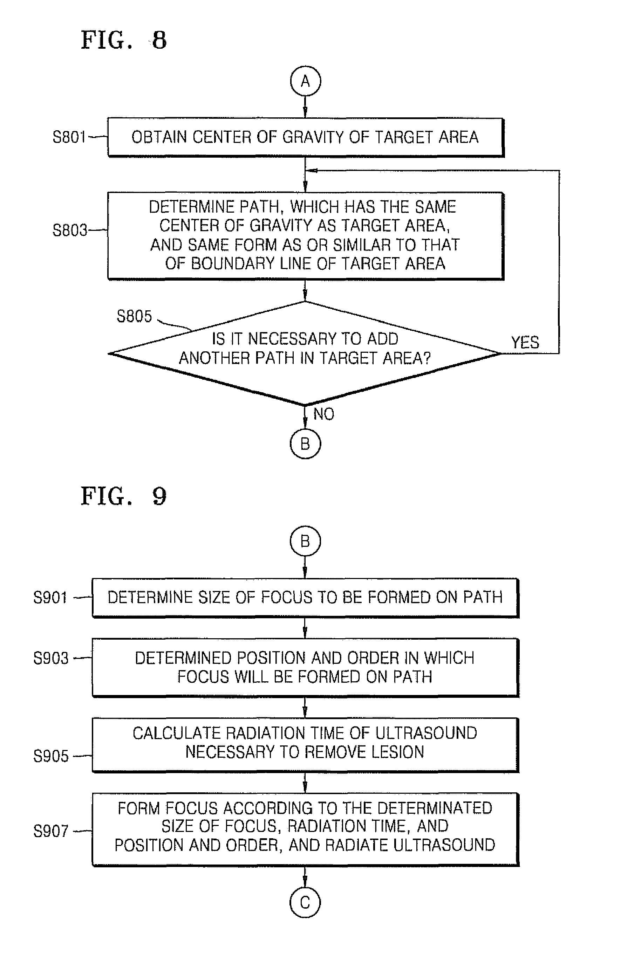

If the target area is received, it is determined whether the target area is an area where a focus may be controlled (operation S703), and, if it is determined that the target area is an area where a focus may not be controlled, a new target area is received again by returning to the operation S701. If it is determined that the target area is an area where a focus may be controlled, in operation S705, a path along which a focus will move in the target area is determined depending on a form of the target area. The operation S705 is illustrated in detail in FIG. 8. Referring to FIG. 8, the center point of gravity of the target area is obtained (operation S801), and a path, which includes the center point of gravity, which is the same as that of the target area and a form of which is the same as or similar to that of a boundary line of the target area, is determined (operation S803). Since a method of determining the path of which form is the same as or similar to that of the boundary line of the target area has been explained in relation to FIG. 5, a detailed explanation is omitted. If the path is set in the operation S803, it is determined whether it is necessary to add another path in the target area (operation S805). If it is determined that it is necessary, another path is set again by returning to the operation S803, and if it is determined that this is not necessary, an operation of determining a path is ended. It is determined where it is necessary to add another path in the object area in consideration of a form of the target area and a size of a focus to be used.

If the path is determined, in operation S707 of FIG. 7, a focus is formed on the determined path, and ultrasound radiation is performed. The operation S707 is illustrated in detail in FIG. 9. Referring to FIG. 9, if the path along which the focus will move is determined in operation S705 of FIG. 7, the size of the focus to be formed on the path is determined (operation S901). A position and order in which the focus will be formed on the path is determined in consideration of the determined size of the focus (operation S903), and a radiation time of ultrasound, which is necessary to remove a lesion mass, is calculated by using the determined path, the size of the focus, and the position where the focus will be formed (operation S905). Since a method of calculating the radiation time by using the Pennes' bioheat transfer equation and the Sapareto and Dewey equation has been described in an explanation of FIG. 1, a detailed explanation is omitted. In addition, ultrasound is radiated depending on the determined path, the size of the focus, and the position and order in which the focus will be formed (operation S907).

Referring back to FIG. 7, if the radiation of ultrasound is performed in the operation S707, it is monitored whether the lesion mass is removed (operation S709). If the lesion mass is not removed, ultrasound radiation is further performed returning to the operation S707. If the lesion mass is removed, all processes of the method of controlling the focus are ended.

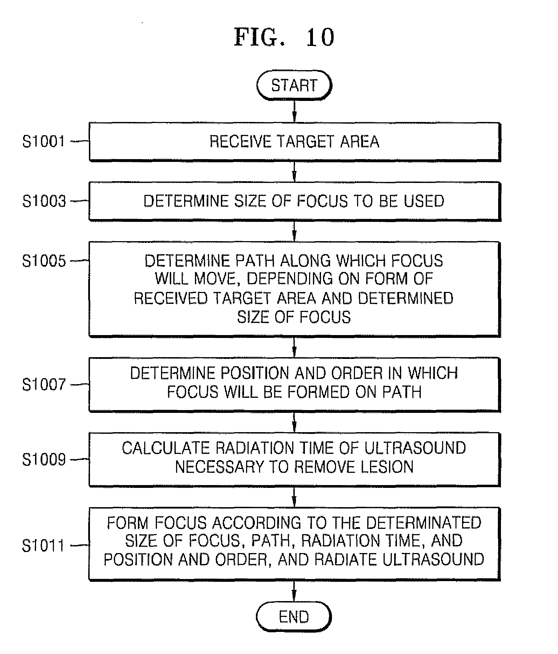

FIG. 10 is a flowchart for explaining a method of controlling a focus in the focused ultrasound therapy apparatus according to an embodiment. Referring to FIG. 10, a target area is received (operation S1001), and a size of a focus to be used is determined first before determining a path along which the focus will move (operation S1003). Next, the path along which the focus will move is determined depending on a form of the received target area and the determined size of the focus (operation S1005). That is, the path along which the focus will move is determined to be the same as or similar to that of a boundary line of the target area. In the case of setting a plurality of paths, a distance between the plurality of paths is determined depending on the size of the focus to be formed on each path. Next, a position and order in which the focus will be formed on each path is determined (operation S1007), and a radiation time of ultrasound, which is necessary to remove a lesion, is calculated (operation S1009). Thereafter, the focus is formed depending on the size of the focus, the path, the radiation time, and the position and order, which are determined in the above operations, and then ultrasound is radiated to the focus (operation S1011).

Processes, functions, methods, and/or software in apparatuses described herein may be recorded, stored, or fixed in one or more non-transitory computer-readable storage (recording) media that includes program instructions (computer-readable instructions) to be implemented by a computer to cause one or more processors to execute or perform the program instructions. The media may also include, alone or in combination with the program instructions, data files, data structures, and the like. The media and program instructions may be those specially designed and constructed, or they may be of the kind well-known and available to those having skill in the computer software arts. Examples of non-transitory computer-readable storage media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The described hardware devices may be configured to act as one or more software modules that are recorded, stored, or fixed in one or more computer-readable storage media, in order to perform the operations and methods described above, or vice versa. In addition, non-transitory computer-readable storage media may be distributed among computer systems connected through a network and computer-readable codes or program instructions may be stored and executed in a decentralized manner. In addition, the computer-readable storage media may also be embodied in at least one application specific integrated circuit (ASIC) or Field Programmable Gate Array (FPGA).

Although a few embodiments have been shown and described, it would be appreciated by those skilled in the art that changes may be made in these embodiments without departing from the principles and spirit of the disclosure, the scope of which is defined in the claims and their equivalents.

**Claims**

1.  A method of controlling a focus in a focused ultrasound therapy apparatus for radiating ultrasound to remove a lesion, the method comprising:

    receiving a target area to which ultrasound is radiated to remove the lesion;
    determining a path along which the focus moves in the target area, depending on a form of the target area; and
    forming the focus on the determined path and then radiating ultrasound to the target area.

2.  The method of claim 1, further comprising determining a size of the focus to be used for ultrasound radiation, wherein the determining of the path includes determining the path along which the focus moves in the target area, depending on the determined size of the focus.

3.  The method of claim 1 or 2, wherein the determining of the path includes setting a path having a form which is the same as a boundary line of the target area.

4.  The method of any of claim 1-3, wherein the determining of the path comprises setting more than two paths, and the radiating of ultrasound comprises forming focuses having sizes different from each other on paths different from each other and then radiating ultrasound.

5.  The method of any of claims 1-4, further comprising forming a multi-focus and then radiating ultrasound to the target area.

6.  The method of any of claims 1-5, wherein the focus in a portion close to an edge of the target area is smaller than the focus in another portion further removed from the edge of the target area.

7.  The method of any of claims 1-6, wherein the radiating ultrasound to the target area comprises:

    calculating a radiation time of ultrasound; and
    radiating ultrasound to the target area for the calculated radiation time.

8.  The method of claim 7, wherein the calculating of the radiation time includes estimating a temperature change of the target area depending on time, in a case of radiating ultrasound to the target area, by using a distance from a point where ultrasound is generated from the target area, a mass composition of an organ, and a blood diffusion degree in the body, calculating a time during which an energy transmitted to the target area reaches a value higher than a critical value by using the estimated temperature change, and then determining the calculated time as the radiation time.

9.  The method of any of claims 1-8, wherein the forming the focus on the determined path and then the radiating of ultrasound comprises:

    determining positions where the focus is formed on the determined path; and
    determining an order of the determined positions;
    forming the focus depending on the determined order; and
    then radiating ultrasound.

10. The method of any of claims 1-9, further comprising:

    determining whether control of the focus in the target area is possible after receiving the target area; and
    receiving another target area if control of the focus in the target area is impossible.

11. A focused ultrasound therapy apparatus for radiating ultrasound to remove a lesion, the apparatus comprising:

    a target area input unit to receive a target area to which ultrasound is radiated to remove the lesion;
    a path determination unit to determine a path along which a focus moves in the target area, depending on a form of the target area;
    an ultrasound converter to receive an electrical signal and then to generate ultrasound; and
    a focus controller to control the ultrasound converter so as to form the focus on the determined path and then radiate ultrasound to the target area.

**12.** The apparatus of claim 11, wherein the path determination unit sets more than two paths, and the focus control unit controls the ultrasound converter so as to form focuses having sizes different from each other on paths different from each other and then radiate ultrasound.

**13.** The apparatus of claim 11 or 12, wherein the focus control unit controls the ultrasound converter so as to form a multi-focus and then radiate ultrasound.

**14.** The apparatus of claim 11-13, further comprising a radiation time calculator to calculate a radiation time of ultrasound, wherein the focus controller controls the ultrasound converter so as to radiate ultrasound to the target area for the radiation time calculated in the radiation time calculator,
wherein the radiation time calculator preferably estimates a temperature change of the target area depending on time, in the case of radiating the ultrasound to the target area, by using a distance from a point, in which the ultrasound is generated, from the target area, an organization composition of an organ, and a blood diffusion degree in the body, calculates a time during which an energy transmitted to the target area reaches a value higher than a critical value by using the estimated temperature change, and then determines the calculated time as the radiation time.

**15.** A non-transitory computer-readable recording medium storing computer readable instructions that control at least one processor to implement the method of any of claims 1-10.

FIG. 1

# FIG. 2

FIG. 3A

FIG. 3B

FIG. 3C

FIG. 4

FIG. 5A

FIG. 5B

FIG. 5C

FIG. 6

# FIG. 7

```
          ( START )
             │
             ▼
S701 ── [ RECEIVE TARGET AREA ] ◄──────┐
             │                         │
             ▼                         │
S703      ╱ IS IT ╲                    │
        ╱ POSSIBLE TO CONTROL ╲  NO    │
        ╲ FOCUS IN TARGET ╱ ──────────┤
          ╲ AREA? ╱                    │
             │ YES                     │
             ▼                         │
            (A)                        │
             │                         │
             ▼                         │
      ┌─────────────────────────┐      │
S705 ─┤ DETERMINE PATH ALONG WHICH│    │
      │ FOCUS WILL MOVE, DEPENDING ON│ │
      │ FORM OF TARGET AREA      │      │
      └─────────────────────────┘      │
             │                         │
             ▼                         │
            (B) ◄────────────────┐     │
             │                   │     │
             ▼                   │     │
      ┌─────────────────────────┐│     │
S707 ─┤ FORM FOCUS ALONG DETERMINED││   │
      │ PATH AND PERFORM ULTRASOUND││   │
      │ RADIATION               ││     │
      └─────────────────────────┘│     │
             │                   │     │
             ▼                   │     │
            (C)                  │     │
             │                   │     │
             ▼                   │     │
S709      ╱ ╲                    │     │
        ╱ IS LESION ╲   NO       │     │
        ╲ REMOVED? ╱ ────────────┘     │
          ╲ ╱                          
             │ YES
             ▼
          ( END )
```

# FIG. 8

(A)

S801 — OBTAIN CENTER OF GRAVITY OF TARGET AREA

S803 — DETERMINE PATH, WHICH HAS THE SAME CENTER OF GRAVITY AS TARGET AREA, AND SAME FORM AS OR SIMILAR TO THAT OF BOUNDARY LINE OF TARGET AREA

S805 — IS IT NECESSARY TO ADD ANOTHER PATH IN TARGET AREA? — YES

NO

(B)

# FIG. 9

(B)

S901 — DETERMINE SIZE OF FOCUS TO BE FORMED ON PATH

S903 — DETERMINED POSITION AND ORDER IN WHICH FOCUS WILL BE FORMED ON PATH

S905 — CALCULATE RADIATION TIME OF ULTRASOUND NECESSARY TO REMOVE LESION

S907 — FORM FOCUS ACCORDING TO THE DETERMINATED SIZE OF FOCUS, RADIATION TIME, AND POSITION AND ORDER, AND RADIATE ULTRASOUND

(C)

## FIG. 10

START

S1001 — RECEIVE TARGET AREA

S1003 — DETERMINE SIZE OF FOCUS TO BE USED

S1005 — DETERMINE PATH ALONG WHICH FOCUS WILL MOVE, DEPENDING ON FORM OF RECEIVED TARGET AREA AND DETERMINED SIZE OF FOCUS

S1007 — DETERMINE POSITION AND ORDER IN WHICH FOCUS WILL BE FORMED ON PATH

S1009 — CALCULATE RADIATION TIME OF ULTRASOUND NECESSARY TO REMOVE LESION

S1011 — FORM FOCUS ACCORDING TO THE DETERMINATED SIZE OF FOCUS, PATH, RADIATION TIME, AND POSITION AND ORDER, AND RADIATE ULTRASOUND

END

Europäisches
Patentamt

European
Patent Office

Office européen
des brevets

## EUROPEAN SEARCH REPORT

Application Number

EP 12 17 4298

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/122449 A1 (KONINKL PHILIPS ELECTRONICS NV [NL]; RAJU BALASUNDARA [US]; SOKKA SHUN) 28 October 2010 (2010-10-28) * page 8, line 22 - page 10, line 25 * * page 19, lines 16-34; figure 7 * | 11,12,14 | INV. A61N7/02 |
| X | US 2006/058671 A1 (VITEK SHUKI [IL] ET AL) 16 March 2006 (2006-03-16) * paragraphs [0029] - [0032], [0035], [0038], [0049] * | 11,13,14 | |
| X | US 6 618 620 B1 (FREUNDLICH DAVID [IL] ET AL) 9 September 2003 (2003-09-09) * column 3, line 9 - column 4, line 58 * * column 7, line 15 - column 10, line 67; figures 5,8 * | 11,12,14 | |
| X | US 2007/219448 A1 (SEIP RALF [US] ET AL) 20 September 2007 (2007-09-20) * paragraphs [0038], [0042], [0045], [0106], [0121], [0124]; figures 10A-B,14 * | 11-14 | TECHNICAL FIELDS SEARCHED (IPC) A61N A61B |
| X | US 2010/022921 A1 (SEIP RALF [US] ET AL) 28 January 2010 (2010-01-28) * paragraphs [0047] - [0050], [0079] - [0080]; figure 2 * | 11-14 | |
| X | US 5 873 845 A (CLINE HARVEY ELLIS [US] ET AL) 23 February 1999 (1999-02-23) * column 2, line 65 - column 4, line 37; figure 4B * | 11,13,14 | |
| | -/-- | | |

The present search report has been drawn up for all claims

| Place of search | Date of completion of the search | Examiner |
|---|---|---|
| Munich | 16 November 2012 | Link, Tatiana |

EPO FORM 1503 03.82 (P04C01)

EUROPEAN SEARCH REPORT

Application Number

EP 12 17 4298

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/080631 A2 (KONINKL PHILIPS ELECTRONICS NV; RADULESCU EMIL G; EHNHOLM GOSTA JAKOB;) 7 July 2011 (2011-07-07) * page 3, line 13 - page 4, line 10; figure 5 * | 11,13,14 | |
| X | US 6 086 535 A (ISHIBASHI YOSHIHARU [JP] ET AL) 11 July 2000 (2000-07-11) * column 15, lines 47-67 * * column 16, lines 20-31 * * column 27, lines 23-55 * * column 35, lines 46-50; figures 35,36 * | 11,14 | |

TECHNICAL FIELDS
SEARCHED      (IPC)

The present search report has been drawn up for all claims

| Place of search | Date of completion of the search | Examiner |
|---|---|---|
| Munich | 16 November 2012 | Link, Tatiana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 17 4298

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2012

| Patent document cited in search report | | Publication date | Patent family member(s) | | Publication date |
|---|---|---|---|---|---|
| WO 2010122449 | A1 | 28-10-2010 | CN | 102405078 A | 04-04-2012 |
| | | | EP | 2421610 A1 | 29-02-2012 |
| | | | KR | 20120088545 A | 08-08-2012 |
| | | | US | 2012035464 A1 | 09-02-2012 |
| | | | WO | 2010122449 A1 | 28-10-2010 |
| US 2006058671 | A1 | 16-03-2006 | EP | 1786518 A1 | 23-05-2007 |
| | | | JP | 4820820 B2 | 24-11-2011 |
| | | | JP | 2008509713 A | 03-04-2008 |
| | | | US | 2006058671 A1 | 16-03-2006 |
| | | | WO | 2006018686 A1 | 23-02-2006 |
| US 6618620 | B1 | 09-09-2003 | AU | 1846202 A | 11-06-2002 |
| | | | CN | 1477985 A | 25-02-2004 |
| | | | EP | 1349612 A1 | 08-10-2003 |
| | | | JP | 4080332 B2 | 23-04-2008 |
| | | | JP | 2004514510 A | 20-05-2004 |
| | | | US | 6618620 B1 | 09-09-2003 |
| | | | WO | 0243804 A1 | 06-06-2002 |
| US 2007219448 | A1 | 20-09-2007 | EP | 1755458 A2 | 28-02-2007 |
| | | | US | 2007219448 A1 | 20-09-2007 |
| | | | US | 2012150035 A1 | 14-06-2012 |
| | | | WO | 2005107601 A2 | 17-11-2005 |
| US 2010022921 | A1 | 28-01-2010 | US | 2005240127 A1 | 27-10-2005 |
| | | | US | 2010022921 A1 | 28-01-2010 |
| US 5873845 | A | 23-02-1999 | NONE | | |
| WO 2011080631 | A2 | 07-07-2011 | CN | 102711914 A | 03-10-2012 |
| | | | EP | 2519321 A2 | 07-11-2012 |
| | | | WO | 2011080631 A2 | 07-07-2011 |
| US 6086535 | A | 11-07-2000 | DE | 69634714 D1 | 16-06-2005 |
| | | | DE | 69634714 T2 | 19-01-2006 |
| | | | EP | 0734742 A2 | 02-10-1996 |
| | | | US | 5984881 A | 16-11-1999 |
| | | | US | 6086535 A | 11-07-2000 |
| | | | US | 6267734 B1 | 31-07-2001 |
| | | | US | 6280402 B1 | 28-08-2001 |
| | | | US | 6454713 B1 | 24-09-2002 |

EPO FORM P0459

For more details about this annex : see Official Journal of the European Patent Office, No. 12/82